# EUROPÄISCHE PATENTSCHRIFT

(11) **EP 3 024 374 B1**
(45) Veröffentlichungstag und Bekanntmachung des Hinweises auf die Patenterteilung: **23.05.2018**
(21) Anmeldenummer: 14741617.6
(22) Anmeldetag: 21.07.2014
(51) Int. Cl.: A61B 1/00

(54) **ABDICHTUNGSBAUTEIL FÜR EINEN ENDOSKOPSTECKER**
SEALING COMPONENT FOR AN ENDOSCOPE CONNECTOR
ÉLÉMENT D'ÉTANCHÉITÉ POUR CONNECTEUR D'ENDOSCOPE

(30) Priorität: 22.07.2013 DE 102013214278
(43) Veröffentlichungstag der Anmeldung: 01.06.2016
(73) Patentinhaber: Digital Endoscopy GmbH, 86316 Friedberg (DE)
(72) Erfinder: RILLI, Christoph, 12055 Berlin (DE)
(74) Vertreter: TBK
(86) Internationale Anmeldenummer: PCT/EP2014/065587
(87) Internationale Veröffentlichungsnummer: WO 2015/011075

(56) Entgegenhaltungen:
- WO-A1-2011/108157
- JP-A- 2003 190 085
- US-A- 4 404 963
- US-A- 5 630 419
- US-B2- 7 841 880
- Anonymous: "Products | BMP-TAPPI ", , 30 June 2013 (2013-06-30), XP055394249, Retrieved from the Internet: URL:https://web.archive.org/web/2013063008 2009/http://www.bmp-tappi.com:80/products [retrieved on 2017-07-27]
- Anonymous: "10. Tappo per innesti rapidi femmina", , 22 June 2013 (2013-06-22), XP055394266, Retrieved from the Internet: URL:https://web.archive.org/web/2013062216 1734/http:/www.bmp-tappi.it:80/portfolio_i tem/tappo-per-innesti-rapidi-femmina [retrieved on 2017-07-27]

## Beschreibung

Die vorliegende Erfindung bezieht sich auf ein Endoskop mit einem Endoskopkabel, das einen Endoskopstecker hat, und einem Abdichtungsbauteil für den Endoskopstecker.

Ein übliches Endoskop ist mittels Endoskopkabel zur Datenverbindung mit einer Auswerteeinheit verbunden. Das Endoskopkabel hat einen Anschlussstecker, der in die Auswerteeinheit eingesteckt und ausgesteckt werden kann.

Endoskope müssen nach ihrer Verwendung gereinigt werden. Für die Reinigung eines Endoskops wird das Endoskopkabel aus der Auswerteeinheit herausgezogen. Dann muss auf den Anschlussstecker eine Dichtungskappe aufgesetzt werden, damit die Anschlüsse geschützt bleiben.

### Stand der Technik

US 7 841 880 B2 lehrt für ein Endoskop eine wasserdichte Kappe, die über einen drahtartigen Körper an einem Endoskopstecker angebunden ist.

EP 0 028 396 B1 lehrt für ein Endoskop eine zylindrische Kappe, die von einem Endoskopverbindungsstück separat ist. Die zylindrische Kappe ist ein separates Einzelteil.

DE 101 48 099 B4 lehrt eine wasserdichte Abschlusskappe für ein Endoskop, die vom Endoskop separat vorgesehen ist. Die wasserdichte Abschlusskappe ist ein separates Einzelteil.

US 6 547 722 B1 lehrt ebenfalls eine wasserdichte Abschlusskappe für ein Endoskop. Die Abschlusskappe ist am Endoskopverbindungsstück mit einem drahtartigen Gebilde angebunden.

US 20020115907 A1 lehrt ein Endoskop mit wasserdichter Kappe. Die Kappe ist am Endoskopverbindungsstück mit einer Kette angebunden.

Bisherige Endoskope haben also eine vom abzudichtenden Körper völlig separate Kappe oder eine am abzudichtenden Körper per Kabel, Draht, Kette etc. angebundene Kappe.

Die Kappe und die Kette, das Kabel oder der Draht sind schwer zu reinigen, da es viele Hinterschnitte gibt. Hinzu kommt, dass die Kette, das Kabel oder der Draht reißen kann und die Kappe verlorengeht. Dann könnte keine Reinigung mehr vorgenommen werden kann.

Die Aufgabe der vorliegenden Erfindung ist es, ein verbessertes Endoskop zu schaffen.

Diese Aufgabe ist durch ein Endoskop gemäß Anspruch 1 gelöst.

Weiterentwicklungen sind Gegenstand der abhängigen Ansprüche.

Gemäß Anspruch 1 hat das Endoskop das Abdichtungsbauteil.

Ein solches Abdichtungsbauteil ist somit mit dem Endoskopkabel integrierbar und geht beim Reinigungsvorgang nicht verloren.

Die Endoskopsteckereinführöffnung des Körpers kann mit einem Dichtungselement versehen sein. Dadurch kann das Steckeranschlussende gut abgedichtet in die Endoskopsteckereinführöffnung des Abdichtungsbauteils eingesteckt werden. Beim Reinigungsvorgang sorgt das Dichtungselement dafür, dass die Reinigungsflüssigkeit nicht an die Anschlüsse des Steckeranschlussendes gelangt.

Der Körper des Abdichtungsbauteils kann aus Kunststoff hergestellt sein. Der Körper des Abdichtungsbauteils ist dadurch kostengünstig und einfach herstellbar.

Der Befestigungsabschnitt kann eine Durchgangsöffnung aufweisen, wobei in der Durchgangsöffnung das Endoskopkabel verschiebefähig befestigbar ist. Ein solches Abdichtungsbauteil ist mit dem Endoskopkabel sicher verbunden, kann aber seine Position am Endoskopkabel durch Verschieben verändern. So kann das Abdichtungsbauteil am Anfangsende des Endoskopkabels, also in der Nähe einer Griffeinheit positioniert werden und bei Bedarf zum entgegengesetzten Ende, also in die Nähe des Endoskopsteckers geschoben werden. Die Durchgangsöffnung kann so gewählt werden, dass das Abdichtungsbauteil lose am Endoskopkabel sitzt. In einem solchen Fall hat die Durchgangsöffnung des Abdichtungsbauteils einen Innendurchmesser, der ausreichend größer als der Außendurchmesser des Endoskopkabels ist. Die Durchgangsöffnung kann aber auch so gewählt werden, dass das Abdichtungsbauteil relativ straff am Endoskopkabel sitzt. In einem solchen Fall hat die Durchgangsöffnung des Abdichtungsbauteils einen Innendurchmesser, der fast gleich groß wie der Außendurchmesser des Endoskopkabels ist.

Der Befestigungsabschnitt kann am Endoskopkabel positionsunveränderbar befestigbar sein. Beispielsweise kann das Abdichtungsbauteil am Endoskopkabel angegossen oder angespritzt werden.

Der Befestigungsabschnitt kann eine Klemmeinrichtung aufweisen, wobei die Klemmeinrichtung am Endoskopkabel anklemmbar ist. Eine solche Klemmeinrichtung sorgt für eine sichere Befestigung des Abdichtungsbauteils am Endoskopkabel, wobei dennoch das Abdichtungsbauteil vom Endoskopkabel entfernt werden kann. In diesem Fall kann das Abdichtungsbauteil separat gereinigt werden. Außerdem ist ein besonders schnelles Auswechseln eines z.B. defekten Abdichtungsbauteils durch ein anderes Abdichtungsbauteil möglich. Das Endoskop kann weiter eine Bedieneinheit aufweisen, wobei am zur Bedieneinheit entgegengesetzten Ende des Endoskopkabels der Endoskopstecker angeordnet ist. Das Abdichtungsbauteil sitzt am Endoskopkabel. Ein Steckeranschlussende des Endoskopsteckers ist in die sacklochartige Endoskopsteckereinführöffnung des Abdichtungsbauteils einsteckbar, wobei im eingesteckten Zustand das Abdichtungsbauteil das Steckeranschlussende des Endoskopsteckers abdichtet.

Das Abdichtungsbauteil ist dabei am Endoskopkabel befestigt. Ein solches Endoskop kann einfach und sicher gereinigt werden, da der Endoskopstecker einfach in das am Endoskopkabel sitzende Abdichtungsbauteil eingesteckt wird. Da das Abdichtungsbauteil am Endoskopkabel sitzt, geht es beim Reinigen nicht verloren.

Das Abdichtungsbauteil kann am Befestigungsabschnitt eine Durchgangsöffnung aufweisen, wobei in der Durchgangsöffnung das Endoskopkabel unter Haftreibung sitzt und verschiebbar ist. Hierbei ist das Abdichtungsbauteil besonders sicher mit dem Endoskopkabel verbunden.

Der Befestigungsabschnitt des Abdichtungsbauteils kann am Endoskopkabel integriert sein. Hierbei kann das Abdichtungsbauteil am Endoskopkabel angegossen oder angespritzt sein. Um den Mantel des Endoskopkabels wird dabei die Form des Abdichtungsbauteils z.B. durch ein Kunststoffspritzverfahren, Kunststoffdruckgussverfahren, Zweistufen-Formverfahren, etc. erzeugt. Ein anderes geeignetes Herstellverfahren kann ebenfalls zur Anwendung gelangen, durch das das Abdichtungsbauteil auf das Endoskopkabel aufgebracht werden kann.

Der Befestigungsabschnitt kann am Endoskopkabel angeformt sein. Das Abdichtungsbauteil und das Endoskopkabel können aus dem gleichen Material bestehen und ein einem Fertigungsvorgang gleichzeitig hergestellt sein. Das Abdichtungsbauteil und das Endoskopkabel sind dann nicht mehr trennbar.

Der Befestigungsabschnitt und der Mantel des Endoskopkabels können einstückig sein. Hierbei ist das Abdichtungsbauteil mit dem Endoskopkabel einstückig, d.h. als eine Einheit gestaltet.

Der Befestigungsabschnitt des Abdichtungsbauteils kann eine Klemmeinrichtung aufweisen, wobei die Klemmeinrichtung am Endoskopkabel angeklemmt ist.

Die Klemmeinrichtung befestigt das Abdichtungsbauteil sicher am Endoskopkabel, wobei aber das Abdichtungsbauteil bei Bedarf vom Endoskopkabel wieder entfernt werden könnte. In diesem Fall kann das Abdichtungsbauteil separat gereinigt werden. Außerdem ist ein besonders schnelles Auswechseln eines z.B. defekten Abdichtungsbauteils durch ein anderes Abdichtungsbauteil möglich. Vorhandene Endoskope können mit einem solchen Abdichtungsbauteil nachgerüstet werden, indem das neue Abdichtungsbauteil am alten Endoskopkabel angeklemmt wird.

An dem Bereich des Endoskopkabels, der am Befestigungsabschnitt angrenzt, kann ein Knickschutz vorgesehen sein, der ein Knicken des Endoskopkabels in der Nähe des Abdichtungsbauteils verhindert. Ein solcher Knickschutz kann sogar beispielsweise bei einer Bauweise angewendet werden, in der ein Endoskopkabel an der Position, an der das Abdichtungsbauteil vorgesehen werden soll, aufgeschnitten wird und an beiden Seiten des Abdichtungsbauteils wieder befestigt wird. Bei einem solchen Endoskop kann das Abdichtungsbauteil als Knickschutz an der proximalen und an der distalen Seite einen zylindrischen oder sich verjüngenden Vorsprung mit einem derartigen Außenmass, dass der jeweilige Vorsprung in das jeweilige Ende des Endoskopkabels, das sich nach dem Durchtrennen des Endoskopkabels ergibt, eingesteckt werden kann.

Anstelle des Vorsprungs kann auch ein entsprechendes vorragendes zylindrisches oder sich verjüngendes Rohr in jeweils vorbestimmter Länge an der proximalen und an der distalen Seite des Abdichtungsbauteils vorgesehen sein.

Als Knickschutz kann auch eine am Aussenumfang des Endoskopkabels anliegend angeordnete Spiralfeder vorgesehen sein, die am Befestigungsabschnitt des Abdichtungsbauteils jeweils an der distalen und an der proximalen Seite verankert ist.

Dadurch wird in technisch einfacher, aber wirkungsvoller Weise ein Abknicken des Endoskopkabels an der Übergangsstelle zum Abdichtungsbauteil verhindert.

Die erörterten Merkmale können geeignet kombiniert werden.

Kurzbeschreibung der Zeichnungen
Fig. 1 zeigt ein Endoskop mit einem Abdichtungsbauteil gemäß der vorliegenden Erfindung in einem Zustand, bei dem ein Endoskopstecker in das Abdichtungsbauteil eingesteckt ist.
Fig. 2 zeigt das Endoskop mit dem Abdichtungsbauteil gemäß der vorliegenden Erfindung in einem Zustand, bei dem ein Endoskopstecker aus dem Abdichtungsbauteil herausgenommen ist.
Fig. 3 zeigt ein Abdichtungsbauteil eines weiteren Ausführungsbeispiels gemäß der vorliegenden Erfindung ohne das Endoskop.

Nachstehend ist die vorliegende Erfindung detailliert unter Bezugnahme auf Ausführungsbeispiele anhand der vorliegenden Zeichnungen beschrieben.

### Ausführungsbeispiel

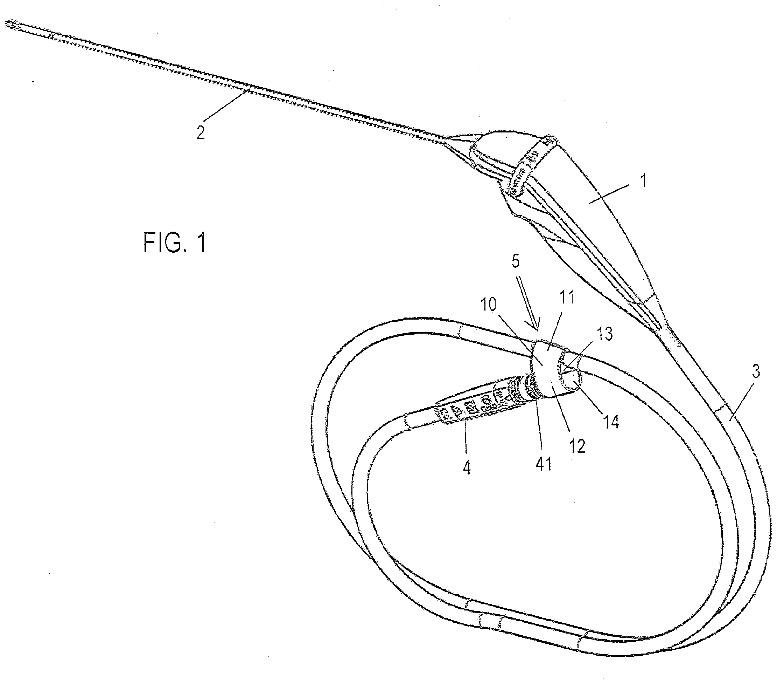

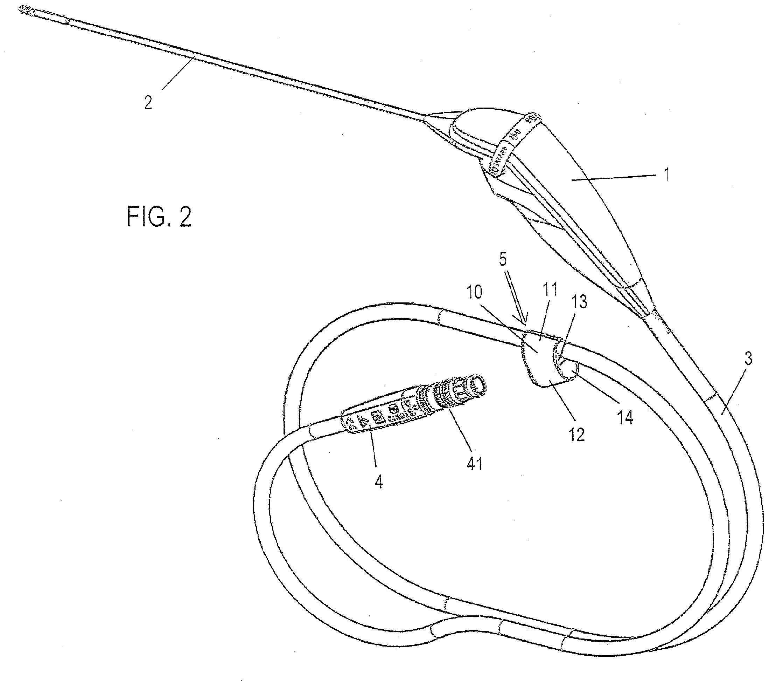

Zunächst ist ein Ausführungsbeispiel gemäß den Figuren 1 und 2 beschrieben.

Fig. 1 zeigt ein Endoskop mit einem Abdichtungsbauteil gemäß der vorliegenden Erfindung in einem Zustand, bei dem ein Endoskopstecker in das Abdichtungsbauteil eingesteckt ist, und Fig. 2 zeigt ein Endoskop mit einem Abdichtungsbauteil gemäß der vorliegenden Erfindung in einem Zustand, bei dem ein Endoskopstecker aus dem Abdichtungsbauteil herausgenommen ist.

Ein Endoskop weist ein Griffstück 1 einer Bedieneinheit und an der distalen Seite von ihr einen Endoskopschlauch 2 auf. Alternativ kann das Endoskop an der distalen Seite des Griffes 1 anstelle des Endoskopschlauches 2 einen Endoskopstab aufweisen.

An der proximalen Seite des Griffstückes 1 der Bedieneinheit weist das Endoskop ein Endoskopkabel 3 auf, das an dem zum Griff 1 entgegengesetzten Ende einen Endoskopstecker 4 hat. Der Endoskopstecker 4 ist mit verschiedenen Betätigungsschaltern etc. versehen. An dem vom Griff 1 entfernten Ende des Endoskopsteckers 4 weist der Endoskopstecker 4 einen elektrischen Anschluss 41 auf, der an eine elektronische Einheit/Auswerteinheit etc. eingesteckt/eingeschraubt werden kann, um die durch das Endoskop gewonnenen Informationen über den Patienten etc. zum Zwecke der Darstellung oder Verarbeitung zu der elektronischen Einheit/Auswerteinheit weiterzuleiten. Im vorliegenden Ausführungsbeispiel weist das Anschlussende 41 des Endoskopsteckers 4 einen Einsteckanschluss auf. Alternativ kann das Anschlussende 41 des Endoskopsteckers 4 ein Gewinde aufweisen.

Das Endoskopkabel 4 weist in der Mitte des Weges zwischen dem Griffstück 1 und dem Endoskopstecker 4 an seinem Außenumfang ein Abdichtungsbauteil 5 auf, wie dies in Fig. 1 gezeigt ist. In dem Ausführungsbeispiel ist dieses Abdichtungsbauteil einstückig mit dem Endoskopkabel 3 vorgesehen. Dabei ist das Abdichtungsbauteil 5 an die Kunststoffschicht an dem Außenumfang des Endoskopkabels 3 durch ein Kunststoffspritzverfahren angespritzt/formgegossen.

Insbesondere weist das Abdichtungsbauteil 5, wie dies in Fig. 1 gezeigt ist, als Hauptelement einen Körper 10 auf, der kegelstumpfartig aufgebaut ist. Das heißt der Körper 10 hat eine in der Darstellung von Fig. 1 an der abgewandten Seite oder der linken Seite in Fig. 1 befindliche Kegelstumpffläche mit einem großen Durchmesser und verjüngt sich entlang der Außenfläche des Körpers 10 in Fig. 1 nach rechts zu einem kleinen Umfang des Kegelstumpfes, d.h. zu einer kleinen Kegelstumpffläche. An diesem kleinen Umfang des Kegelstumpfes, d. h. an der unteren Seite des kegelstumpfartigen Körpers 10 hat das Abdichtungsbauteil einen Unterboden, d.h. die kleine Kegelstumpffläche. Der Unterboden hat eine flache ellipsenartige oder ovale Form. Die ovale oder ellipsenartige Form des Unterbodens, d. h. die Unterbodenfläche ist in zwei annähernd gleich große Abschnitte 13 und 14 geteilt. Die Trennlinie zwischen den beiden Abschnitten 13 und 14 verläuft senkrecht zu der Längsachse des Ovals/der Ellipse des Unterbodens. Der erste Abschnitt 13 des Unterbodens weist eine Öffnung auf, und der zweite Abschnitt 14 des Unterbodens ist vollständig geschlossen.

Dabei bildet der erste Abschnitt 13 des Unterbodens die untere Öffnung eines Durchgangslochs in dem Abdichtungsbauteil, durch die das Endoskopkabel läuft. Der zweite Abschnitt 14 des Unterbodens hingegen ist ausgefüllt und befindet sich an der Seite des Körpers 10 des Abdichtungsbauteils, die zu einer Einstecköffnung für den Endoskopstecker 4 des Abdichtungsbauteils 5 entgegengesetzt ist. Der erste Abschnitt 13 des Unterbodens bildet somit den schlauchseitigen Abschnitt 13 des Unterbodens, und der zweite Abschnitt 14 des Unterbodens des Abdichtungsbauteils 5 bildet somit den endoskopsteckerseitigen Abschnitt 14 des Unterbodens des Abdichtungsbauteils 5.

Der Körper 10 des Abdichtungsbauteils 5 besteht aus einem endoskopschlauchseitigen Teil 11 und einem steckerseitigen Teil 12. Der endoskopschlauchseitige Teil 11 weist den schlauchseitigen Abschnitt 13 des Unterbodens auf. Der steckerseitige Teil 12 weist den endoskopsteckerseitigen Abschnitt 14 des Unterbodens auf. Die Trennfläche zwischen dem endoskopschlauchseitigen Teil 11 und dem steckerseitigen Teil 12 erstreckt sich von der Trennlinie zwischen den beiden Abschnitten 13 und 14 senkrecht zu dieser und teilt den kegelstumpfförmigen Körper 10 in die zwei annähernd gleich große Teile 11 und 12.

In Fig. 1 ist der endoskopschlauchseitige Teil 11 des Abdichtungsbauteils 5 oberhalb angeordnet und ist der einsteckseitige Teil 12 des Abdichtungsbauteils 5 unterhalb angeordnet. An der Seite des Körpers 10 des Abdichtungsbauteils 5, die zum Unterboden (Abschnitt 13 und 14) entgegengesetzt ist, d. h. an der Seite des Kegelstumpfes 10, die den großen Durchmesser aufweist, befindet sich entgegengesetzt zu dem zweiten Abschnitt 14 des Unterbodens eine in Fig. 1 nicht gezeigte sacklochartige Endoskopsteckereinführöffnung, in die der Einsteckanschluss 41 oder Gewindeanschluss 41 des Endoskopsteckers 4 einsteckbar ist. Die Endoskopsteckereinführöffnung des Abdichtungsbauteils 5 hat eine derartige Größe, dass das Anschlussende 41 des Endoskopsteckers 4 passgenau eingesetzt werden kann.

Die Durchgangsöffnung des Abdichtungsbauteils 5 für den Endoskopschlauch 3 und die Endoskopsteckereinführöffnung am Abdichtungsbauteil 5 haben eine derartige Positionsbeziehung, dass die Durchgangsöffnung für das Endoskopkabel 3 und die Endoskopsteckereinführöffnung in einer Ebene liegen und in einem spitzen Winkel zueinander stehen, wie dies in Fig. 1 erkennbar ist. Eine solche Positionsbeziehung liefert eine Konstruktion mit einer vorteilhaften Einsteckrichtung für den Endoskopstecker 4, da das Endoskopkabel 3 mit eingestecktem Endoskopstecker 4 für eine kleine Gesamtgröße sorgt und das Endoskopkabel 3 in einer quasi aufgewickelten/aufgerollten Anordnung ist, wenn der Endoskopstecker 4 in das Abdichtungsbauteil 5 eingesteckt ist.

Am Außenumfang der Endoskopsteckereinführöffnung ist ein zylinderartiges Dichtungselement eingebaut. Das heißt der Außendurchmesser des Dichtungselementes sitzt am Innenumfang der Endoskopsteckereinführöffnung. Die Innenöffnung des Dichtungselementes dient der Aufnahme des Anschlussendes 41 des Endoskopsteckers 4. Das Dichtungselement besteht aus einem geeigneten Dichtungsmaterial, wie beispielsweise Gummi. Das Abdichtungsbauteil 5 ist dagegen aus Kunststoff hergestellt.

Mit Ausnahme der Durchgangsöffnung für das Endoskopkabel 3 und der Einstecköffnung für das Anschlussende 41 des Endoskopsteckers 4, die als ein Sackloch ausgebildet ist, ist der Körper 10 des Abdichtungsbauteils 5 massiv ausgebildet.

Im vorliegenden Ausführungsbeispiel ist der Körper 10 des Abdichtungsbauteils 5 an dem Endoskopkabel 3 des Endoskops so einstückig (integriert) vorgesehen, dass keine Relativverschiebung des Abdichtungsbauteils 5 zum Endoskopkabel 3 möglich ist. Anders ausgedrückt ist das Abdichtungsbauteil 5 an dem Endoskopkabel 3 fest angegossen.

Somit kann gemäß der vorliegenden Erfindung das Anschlussende 41 des Endoskopsteckers 4 sicher in die Endoskopsteckereinführöffnung des Abdichtungsbauteils 5 eingesteckt werden (siehe Fig. 1) und wieder herausgezogen werden (siehe Fig. 2).

Bislang ohne das erfindungsgemäße Abdichtungsbauteil 5 verwendete Endoskope können mit dem Abdichtungsbauteil 5 nachgerüstet werden. Daher kann auch an bei älteren Endoskopen die Erfindung kostengünstig realisiert werden.

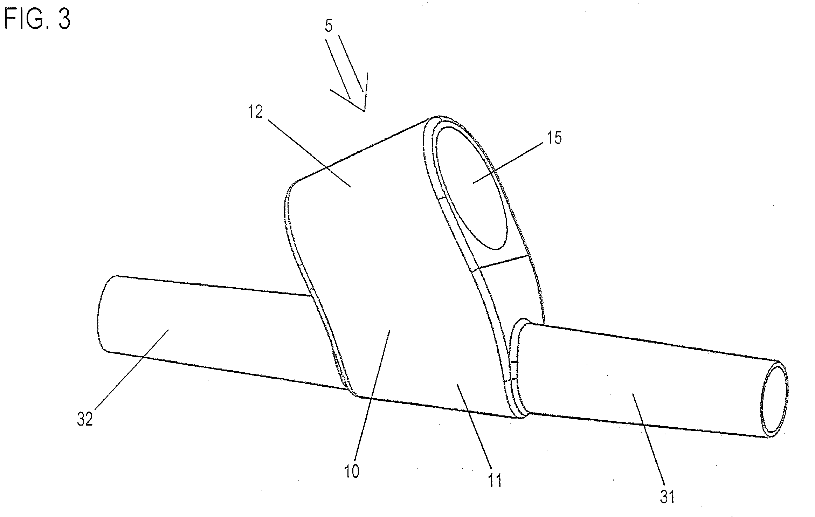

Fig. 3 zeigt ein Abdichtungsbauteil eines weiteren Ausführungsbeispiels gemäß der vorliegenden Erfindung, wobei das Endoskop nicht abgebildet ist. In diesem Ausführungsbeispiel wird ein Abdichtungsbauteil bereitgestellt, das am endoskopschlauchseitigen Teil 11 des Abdichtungsbauteils 5 an der proximalen Seite mit einem sich verjüngenden hohlen Vorsprung 31 und an der distalen Seite mit einem ähnlichen sich verjüngenden hohlen Vorsprung 32 versehen ist. Die Vorsprünge 31 und 32 haben einen derartigen Außendurchmesser, dass ein Endoskopkabel aufgesteckt werden kann.

Somit wird in diesem Ausführungsbeispiel ein existierendes Endoskopkabel (in Fig. 3 nicht gezeigt) durchschnitten, und der jeweilige Vorsprung 31 und 32 wird in das jeweilige Ende des Endoskopkabels, das sich nach dem Durchtrennen des Endoskopkabels ergibt, eingesteckt.

An dem Bereich des Endoskopkabels, der am Befestigungsabschnitt angrenzt, wird dadurch ein Knickschutz vorgesehen, der ein Knicken des Endoskopkabels in der Nähe des Abdichtungsbauteils 5 verhindert. Der Vorsprung 31 dient dabei als distaler Schlauchaufsteckknickschutz 31 und der Vorsprung 32 dient als proximaler Schlauchaufsteckknickschutz 32. Der Vorsprung 32 ragt vom endoskopkabelseitigen Bodenabschnitt 13 des Abdichtungsbauteils 5 vor.

Die Vorsprünge 31 und 32 können am Abdichtungsbauteil 5 angeklebt oder angeschweisst sein. Die Vorsprünge 31 und 32 können auch mit dem Abdichtungsbauteil 5 einstückig gefertigt sein.

Dadurch wird in technisch einfacher, aber wirkungsvoller Weise ein Abknicken des Endoskopkabels 3 an der Stelle, an der das Endoskopkabel 3 am Abdichtungsbauteil 5 montiert ist, verhindert. Das Endoskopkabel 3 wird jeweils auf die Vorsprünge 31 und 32 aufgeschoben. Das Endoskopkabel 3 kann jeweils angeklebt werden, indem vor dem Aufschieben der Enden des Endoskopkabels 3 auf den Vorsprünge 31 und 32 jeweils ein Haftmittel aufgetragen wird.

### Alternativen

Im vorstehend erläuterten Ausführungsbeispiel ist der Körper 10 des Abdichtungsbauteils 5 an dem Endoskopschlauch 3 fest integriert. In einer Alternative ist das Durchgangsloch des Körpers 10 des Abdichtungsbauteils 5 so gestaltet, dass das durch dieses hindurchgeführte Endoskopkabel 3 zu einer Relativbewegung zum Körper 10 des Abdichtungsbauteils 5 entlang der Längsrichtung des Durchgangslochs des Körpers 10 in der Lage ist. Anders ausgedrückt sitzt das Endoskopkabel 3 in der Durchgangsöffnung des Abdichtungsbauteils 5 unter Haftreibung. Das Abdichtungsbauteil 5 ist somit entlang des Endoskopkabels 3, wenn die Haftreibung überwunden ist, verschiebbar. Der Haftreibungskoeffizient, anders ausgedrückt die Größe der Durchgangsöffnung, im Abdichtungsbauteil 5 für das Endoskopkabel 3 ist dabei so gewählt, dass dann, wenn keine Kraft auf das Abdichtungsbauteil 5 ausgeübt wird, das Abdichtungsbauteil 5 am Endoskopkabel 3 positionstreu verbleibt, ohne von selbst entlang des Endoskopkabels 3 zu rutschen.

In einer anderen Alternative kann die Durchgangsöffnung des Abdichtungsbauteils 5 auch so groß sein, dass das Endoskopkabel 3 lose in der Durchgangsöffnung des Abdichtungsbauteils 5 liegt.

In einer weiteren Alternative besitzt das Abdichtungsbauteil 5 anstelle der Durchgangsöffnung für das Endoskopkabel 3 eine Klemmeinrichtung, mit der das Abdichtungsbauteil 5 fest an das Endoskopkabel 3 geklemmt wird.

In einer weiteren Alternative kann der Durchmesser der Endoskopsteckereinführöffnung so gewählt werden, dass in die Endoskopsteckereinführöffnung das Anschlussende 41 des Endoskopsteckers 4 so auch ohne Dichtungselement abdichtend einsteckbar ist. Das Dichtungselement des Ausführungsbeispiels kann dann weggelassen werden.

Im vorstehend erläuterten Ausführungsbeispiel ist der Körper 10 des Abdichtungsbauteils 5 am Endoskopschlauch 3 in einer Kegelstumpfform ausgebildet. Die Erfindung ist nicht auf diese Form beschränkt. Im Prinzip kann für das Abdichtungsbauteil 5 eine beliebige geeignete Form gewählt werden, solange das Abdichtungsbauteil 5 am Endoskopschlauch 3 geeignet befestigt ist und das Anschlussende 41 des Endoskopsteckers 4 sicher in die Endoskopsteckereinführöffnung eingesteckt werden kann. So kann das Abdichtungsbauteil 5 beispielsweise als Quader oder in einer Zylinderform ausgebildet sein.

Im vorstehend erläuterten Ausführungsbeispiel ist die Positionsbeziehung der Durchgangsöffnung des Abdichtungsbauteils 5 für den Endoskopschlauch 3 und die Endoskopsteckereinführöffnung am Abdichtungsbauteil 5 so gewählt, dass die Durchgangsöffnung für den Endoskopschlauch 3 und die Endoskopsteckereinführöffnung in einer Ebene liegen und in einem spitzen Winkel zueinander stehen. Diese Positionsbeziehung kann aber auch so gewählt sein, dass die Durchgangsöffnung für den Endoskopschlauch 3 und die Endoskopsteckereinführöffnung in einer Ebene liegen und parallel zueinander ausgerichtet sind. Diese Positionsbeziehung kann indes auch so gewählt sein, dass die Durchgangsöffnung für den Endoskopschlauch 3 und die Endoskopsteckereinführöffnung in verschiedenen Ebenen liegen. Die Durchgangsöffnung für den Endoskopschlauch 3 und die Endoskopsteckereinführöffnung können auch in einem rechten oder stumpfen Winkel zueinander im Abdichtungsbauteil 5 angeordnet sein, ohne die Grundidee der Erfindung zu verlassen.

Die vorstehend erwähnten Alternativen können - soweit technisch möglich und denkbar - geeignet kombiniert werden.

In sämtlichen Ausführungsformen weist das Abdichtungsbauteil 5 eine sacklochartige Endoskopsteckereinführöffnung 15 auf. In einer weiteren Alternative kann die eine sacklochartige Endoskopsteckereinführöffnung 15 auch durch eine entsprechend geformte Abdeckkappe bedeckt werden. Eine solche Abdeckkappe kann als ein Propfen oder anhand einem Scharnier angelenkt ausgeführt sein und die sacklochartige Endoskopsteckereinführöffnung 15 verschließen, solange der Endoskopstecker 4 nicht eingeführt ist, um die sacklochartige Endoskopsteckereinführöffnung 15 vor Verschmutzung zu schützen.

In Fig. 3 sind die sich verjüngenden hohlen Vorsprünge 31 und 32 als Knickschutz am Abdichtungsbauteil 5 vorgesehen. Alternative können die Vorsprünge 31 und 32 massiv, also nicht hohl vorgesehen werden. Die Vorsprünge 31 und 32 können auch zylindrisch vorgesehen sein, müssen aber einen Aussendurchmesser haben, der ein Aufstreifen des Endoskopkabels 3 erlaubt.

In einer weiteren Alternative kann der Knickschutz am Abdichtungsbauteil 5 auch durch einer distale und eine proximale Spiralfeder in jeweils vorbestimmter Länge vorgesehen sein, die am Befestigungsabschnitt des Abdichtungsbauteils jeweils an der distalen und an der proximalen Seite so verankert sind, dass die Spiralfedern dann am Aussenumfang des Endoskopkabels anliegen.

### Bezugszeichenliste

1 Griffstück, Bedieneinheit
2 Endoskopstab, Endoskopschlauch
3 Endoskopkabel
4 Endoskopstecker
5 Abdichtungsbauteil
10 Körper
11 Befestigungsabschnitt zur Befestigung am Endoskopkabel
12 Endoskopsteckereinführabschnitt
13 erster Bodenabschnitt, endoskopkabelseitiger Bodenabschnitt
14 zweiter Bodenabschnitt, endoskopsteckereinführseitiger Bodenabschnitt
15 Endoskopsteckereinführöffnung
31 distaler Schlauchaufsteckknickschutz
32 proximaler Schlauchaufsteckknickschutz
41 Einsteckanschluss, Gewindeanschlussabschnitt

## Patentansprüche

1. Endoskop mit einem Endoskopkabel (3), welches einen Endoskopstecker (4) aufweist, und mit einem Abdichtungsbauteil (5) für den Endoskopstecker (4), wobei am Ende des Endoskopsteckers (4) ein abzudichtendes Stecker-Anschlussende (41) angeordnet ist,
wobei das Abdichtungsbauteil (5) folgendes aufweist:
einen Körper (10) mit
einer sacklochartigen Endoskopsteckereinführöffnung, in die das Stecker-Anschlussende (41) des Endoskopsteckers (4) abdichtend einsteckbar ist, und
einem Befestigungsabschnitt (11) zur Befestigung an dem Endoskopkabel (3), **dadurch gekennzeichnet, dass**
der Befestigungsabschnitt (11) entweder
- eine Durchgangsöffnung im Körper ist, durch die das Endoskopkabel (3) hindurchführbar ist, oder
- ein Vorsprung am Körper ist, auf dem das Endoskopkabel (3) aufsteckbar ist.

2. Endoskop gemäß Anspruch 1, wobei
die sacklochartige Endoskopsteckereinführöffnung des Körpers (10) mit einem Dichtungselement versehen ist.

3. Endoskop gemäß einem der Ansprüche 1 oder 2, wobei
der Körper (10) aus Kunststoff hergestellt ist.

4. Endoskop gemäß einem der Ansprüche 1 bis 3, wobei
in der Durchgangsöffnung des Befestigungsabschnittes (11) das Endoskopkabel (3) verschiebefähig befestigbar ist.

5. Endoskop gemäß einem der Ansprüche 1 bis 3, wobei
der Befestigungsabschnitt (11) am Endoskopkabel (3) positionsunveränderbar befestigbar ist.

6. Endoskop gemäß einem der Ansprüche 1 bis 3, wobei
der Befestigungsabschnitt (11) eine Klemmeinrichtung aufweist, wobei die Klemmeinrichtung am Endoskopkabel (3) anklemmbar ist.

7. Endoskop gemäß einem der Ansprüche 1 bis 6, wobei der Befestigungsabschnitt (11) einen Knickschutz (31, 32) aufweist.

8. Endoskop gemäß einem der Ansprüche 1 bis 7, mit
einer Bedieneinheit (1), wobei
am zur Bedieneinheit (1) entgegengesetzten Ende des Endoskopkabels (3) der Endoskopstecker (4) angeordnet ist.

9. Endoskop gemäß Anspruch 8, wobei
das Abdichtungsbauteil (5) am Befestigungsabschnitt (11) eine Durchgangsöffnung aufweist, wobei in der Durchgangsöffnung das Endoskopkabel (3) unter Haftreibung sitzt und verschiebbar ist.

10. Endoskop gemäß Anspruch 8, wobei
der Befestigungsabschnitt (11) des Abdichtungsbauteils (5) am Endoskopkabel (3) integriert ist.

11. Endoskop gemäß Anspruch 10, wobei
der Befestigungsabschnitt (11) am Endoskopkabel (3) angeformt ist.

12. Endoskop gemäß Anspruch 10, wobei
der Befestigungsabschnitt (11) und der Mantel des Endoskopkabels (3) einstückig sind.

13. Endoskop gemäß Anspruch 8, wobei
der Befestigungsabschnitt (11) des Abdichtungsbauteils (5) eine Klemmeinrichtung aufweist, wobei die Klemmeinrichtung am Endoskopkabel (3) angeklemmt ist.

14. Endoskop gemäß einem der Ansprüche 8 bis 13, wobei
an dem Bereich des Endoskopkabels (3), der am Befestigungsabschnitt (11) angrenzt, ein Knickschutz (31, 32) vorgesehen ist, der ein Knicken des Endoskopkabels (3) in der Nähe des Abdichtungsbauteils (5) verhindert.

## Claims

1. Endoscope with an endoscope cable (3), which has an endoscope plug (4), and with a sealing component (5) for the endoscope plug (4), wherein a plug attachment end (41) to be sealed is arranged at the end of the endoscope plug (4), wherein the sealing component (5) has the following:
a body (10) with
a blind-hole-like endoscope plug insertion opening into which the plug attachment end (41) of the endoscope plug (4) is sealingly insertable, and
a fastening portion (11) for fastening on the endoscope cable (3),
**characterized in that** the fastening portion (11) is either
- a through-opening in the body, through which through-opening the endoscope cable (3) can be guided, or
- a projection on the body, on which projection the endoscope cable (3) can be plugged.

2. Endoscope according to Claim 1, wherein the blind-hole-like endoscope plug insertion opening of the body (10) is provided with a sealing element.

3. Endoscope according to either of Claims 1 and 2, wherein the body (10) is produced from plastic.

4. Endoscope according to one of Claims 1 to 3, wherein the endoscope cable (3) can be fastened slidably in the through-opening of the fastening portion (11).

5. Endoscope according to one of Claims 1 to 3, wherein the fastening portion (11) can be fastened on the endoscope cable (3) so as to be unchangeable in position.

6. Endoscope according to one of Claims 1 to 3, wherein the fastening portion (11) has a clamping mechanism, wherein the clamping mechanism can be clamped onto the endoscope cable (3).

7. Endoscope according to one of Claims 1 to 6, wherein the fastening portion (11) has kink protection means (31, 32).

8. Endoscope according to one of Claims 1 to 7, with an operating unit (1), wherein the endoscope plug (4) is arranged at the end of the endoscope cable (3) remote from the operating unit (1).

9. Endoscope according to Claim 8, wherein the sealing component (5) has a through-opening at the fastening portion (11), wherein the endoscope cable (3) sits with static friction in the through-opening and is displaceable therein.

10. Endoscope according to Claim 8, wherein the fastening portion (11) of the sealing component (5) is integrated on the endoscope cable (3).

11. Endoscope according to Claim 10, wherein the fastening portion (11) is formed integrally on the endoscope cable (3).

12. Endoscope according to Claim 10, wherein the fastening portion (11) and the jacket of the endoscope cable (3) are in one piece.

13. Endoscope according to Claim 8, wherein the fastening portion (11) of the sealing component (5) has a clamping mechanism, wherein the clamping mechanism is clamped onto the endoscope cable (3).

14. Endoscope according to one of Claims 8 to 13, wherein a kink protection means (31, 32) is provided at the region of the endoscope cable (3) adjoining the fastening portion (11), which kink protection means prevents kinking of the endoscope cable (3) in proximity to the sealing component (5) .

## Revendications

1. Endoscope comprenant un câble d'endoscope (3) qui présente un connecteur d'endoscope (4) et comprenant un composant d'étanchéité (5) pour le connecteur d'endoscope (4), une extrémité de raccord de connecteur à étanchéifier (41) étant disposée à l'extrémité du connecteur d'endoscope (4),
le composant d'étanchéité (5) présentant :
un corps (10) avec
une ouverture d'insertion de connecteur d'endoscope de type trou borgne, dans laquelle peut être enfichée de manière hermétique l'extrémité de raccord de connecteur (41) du connecteur d'endoscope (4), et
une portion de fixation (11) pour la fixation au câble d'endoscope (3),
**caractérisé en ce que** la portion de fixation (11) soit
- est une ouverture de passage dans le corps, à travers laquelle le câble d'endoscope (3) peut être guidé, soit
- est une saillie au niveau du corps, sur laquelle le câble d'endoscope (3) peut être enfiché.

2. Endoscope selon la revendication 1, dans lequel l'ouverture d'insertion de connecteur d'endoscope de type trou borgne du corps (10) est pourvue d'un élément d'étanchéité.

3. Endoscope selon l'une quelconque des revendications 1 ou 2, dans lequel le corps (10) est fabriqué en plastique.

4. Endoscope selon l'une quelconque des revendications 1 à 3, dans lequel le câble d'endoscope (3) peut être fixé de manière déplaçable dans l'ouverture de passage de la portion de fixation (11).

5. Endoscope selon l'une quelconque des revendications 1 à 3, dans lequel la portion de fixation (11) peut être fixée au câble d'endoscope (3) de manière fixée en position.

6. Endoscope selon l'une quelconque des revendications 1 à 3, dans lequel la portion de fixation (11) présente un dispositif de serrage, le dispositif de serrage pouvant être serré sur le câble d'endoscope (3).

7. Endoscope selon l'une quelconque des revendications 1 à 6, dans lequel la portion de fixation (11) présente une protection anti-courbure (31, 32).

8. Endoscope selon l'une quelconque des revendications 1 à 7, comprenant
une unité de commande (1),
le connecteur d'endoscope (4) étant disposé à l'extrémité du câble d'endoscope (3) opposée à l'unité de commande (1).

9. Endoscope selon la revendication 8, dans lequel le composant d'étanchéité (5) présente une ouverture de passage au niveau de la portion de fixation (11), le câble d'endoscope (3) reposant avec adhérence par friction dans l'ouverture de passage et pouvant être déplacé.

10. Endoscope selon la revendication 8, dans lequel la portion de fixation (11) du composant d'étanchéité (5) est intégrée au câble d'endoscope (3).

11. Endoscope selon la revendication 10, dans lequel la portion de fixation (11) est façonnée sur le câble d'endoscope (3).

12. Endoscope selon la revendication 10, dans lequel la portion de fixation (11) et l'enveloppe du câble d'endoscope (3) sont réalisées d'une seule pièce.

13. Endoscope selon la revendication 8, dans lequel la portion de fixation (11) du composant d'étanchéité (5) présente un dispositif de serrage, le dispositif de serrage étant serré sur le câble d'endoscope (3).

14. Endoscope selon l'une quelconque des revendications 8 à 13, dans lequel une protection anti-courbure (31, 32) est prévue au niveau de la région du câble d'endoscope (3) qui est adjacente à la portion de fixation (11), laquelle protection anti-courbure empêche une courbure du câble d'endoscope (3) à proximité du composant d'étanchéité (5).
